# EUROPEAN PATENT APPLICATION

(11) **EP 2 529 731 A1**
(43) Date of publication of application: **05.12.2012**
(21) Application number: 12004142.1
(22) Date of filing: 30.05.2012
(51) Int. Cl.: A61K 9/50, A61K 31/075

(54) **Particulate preparation and method for producing the same**

(30) Priority: 01.06.2011 JP 2011123721; 24.06.2011 JP 2011141058; 15.07.2011 JP 2011157010; 08.05.2012 JP 2012106978
(71) Applicant: Nitto Denko Corporation, Ibaraki-shi, Osaka 567-8680 (JP)
(72) Inventor: Konishi, Tatsuya, Ibaraki-shi, Osaka 567-8680 (JP); Asari, Daisuke, Ibaraki-shi, Osaka 567-8680 (JP); Shishido, Takuya, Ibaraki-shi, Osaka 567-8680 (JP); Okazaki, Arimichi, Ibaraki-shi, Osaka 567-8680 (JP); Hori, Mitsuhiko, Ibaraki-shi, Osaka 567-8680 (JP)
(74) Representative: Müller-Boré & Partner Patentanwälte

(57) **Abstract**

The present invention aims to provide a particulate formulation with an effectively controlled dissolution characteristic of a drug even if the average particle diameter is small. The present invention provides a particulate formulation containing drug particles and a first coating layer coating the drug particles and characterized in that the first coating layer contains a water-insoluble polymer, inorganic particles, and/or a lipid component and the lipid component contains a C₁₅ or higher fatty acid.

## Description

### TECHNICAL FIELD

The present invention relates to a particulate formulation and a method for producing a particulate formulation.

### BACKGROUND ART

Conventionally, regarding a pharmaceutical solid formulation, in order to control a dissolution characteristic or the like of a drug, a pharmaceutical raw material powder is granulated by adding a binder, an excipient, etc., or a pharmaceutical raw material powder is coated with a water-insoluble coating substance. For example, Patent Literature 1 discloses drug coated particles coated with a water-insoluble polymer by using a granule coating and granulating apparatus and a production method of the particles in which a water-insoluble polymer in an amount of 0.1 to 2% to the weight of the drug is used and the particle diameter of the formulation is 17 µm.
For example, Patent Literature 2 also discloses that 1 to 5% of titanium oxide is added to a coating layer coating a drug with ethyl cellulose or HPMC to make the size of the formulation particles be 200 to 300 µm.
For example, Patent Literature 3 also discloses that a drug is coated with HPC or ethyl cellulose; that talc, silicon dioxide, or the like is used; and that the particle diameter of a formulation is 161 µm.

For example, Patent Literature 4 also discloses a sustained release fine grain formulation obtained by melting and mixing a drug, polymer compounds (ethyl cellulose and HPMCAS), stearic acid, and ethanol. Although there is no description of formulation particle diameter in Patent Literature 4, a fine granule produced by an ordinary method is generally about 90 µm or larger.
For example, Patent Literature 5 also discloses a particulate formulation obtained by coating particles (particle diameter of 50 to 250 µm) containing a drug with a lipid component and a polymer compound and in Example 4, disclosed are coated drug particles obtained by coating drug-containing particles by spraying a methanol solution containing 2% of a methacrylic acid copolymer (Eudragit L) and 10% of stearic acid.

However, in general, since the surface area of a drug becomes larger as the particle diameter thereof becomes smaller, controlling dissolution of a drug(particularly suppressing dissolution or masking bitterness) while making drug-containing particles very fine, has been very difficult.

A spray drying method has been employed widely as a simple and efficient granulation and powdering method. However, fluidized bed coating has often been employed for coating a drug for dissolution control of the drug. One of the reasons for that is that it is difficult for the spray drying method to give a sufficient thickness to a coating film and it is therefore impossible to obtain a coated particulate formulation with highly controlled dissolution of the drug.

As a coating technique for a drug using a spray drying method, Patent Literature 6 discloses, for example, production of drug particles having a monolayer coating structure and is suppressed in bitter taste by a spray drying method.
However, Patent Literature 6 does not describe a multilayer coating method by employing a spray drying method and is thus insufficient for control of a dissolution characteristic of a drug.

For example, Patent Literature 7 also discloses a method for producing a drug-containing particulate formulation coated with a thin film of an anti-aggregation agent by spraying and bringing a polymer solution containing a drug and an aqueous solution of the anti-aggregation agent into contact with each other in a spray dryer using different nozzles.
However, the method disclosed in Patent Literature 7 has a problem that production of a product with a stable quality is difficult since the solutions with different compositions are simultaneously sprayed with two nozzles. Further, owing to the special configuration having two nozzles, the equipment is costly and there is another problem that it takes a long time and costs for cleaning and maintenance after the use.
Patent Literature 7 does not disclose a method for simply producing a particulate formulation coated with a multilayer coating and highly controlled in drug dissolution by a spray drying method without employing a complicated method of simultaneously spraying two kinds of spraying solutions respectively with separate nozzles.

For example, Patent Literature 8 also discloses an enteric granule obtained by coating crude granules with a first enteric layer and a second enteric layer.
However, the method for producing the enteric granule disclosed in Patent Literature 8 is not a spray drying method but a coating method in a fluidized granulation manner and as coating polymers for the first enteric layer and the second enteric layer, emulsion polymers dispersed in water are employed and cellulose enteric polymers (HPMCAS) are used for the first layer and the second layer (the dissolution pH of HPMCAS to be used for the second layer is lower than the dissolution pH of HPMCAS to be used for the first layer).

### CITATION LIST

### - Patent Literatures

Patent Literature 1: Japanese Kokai Publication 2008-013480
Patent Literature 2: Japanese Patent No. 3317444
Patent Literature 3: Japanese Kokai Publication 2008-81448
Patent Literature 4: Japanese Kokai Publication Hei-09-020663
Patent Literature 5: Japanese Patent No. 3247511
Patent Literature 6: Japanese Patent No. 3415835
Patent Literature 7: Japanese Patent No. 3205884
Patent Literature 8: Japanese Kokai Publication 2007-332101

### SUMMARY OF INVENTION

### - Technical Problem

In view of the above state of the art, it is an object of the present invention is to provide a particulate formulation with an effectively controlled dissolution characteristic of a drug even if the average particle diameter is small and a method for producing a particulate formulation with highly controlled dissolution of a drug: and the method is capable of simply producing a particulate formulation by coating particles of a drug with a multilayer coating by a spray drying method without employing a complicated method of simultaneously spraying two kinds of spraying solutions with different nozzles.

### - Solution to Problem

In order to solve the above-mentioned problem, the present inventors made various investigations, have found that dissolution of a drug can preferably be controlled by using a water-insoluble polymer, inorganic particles, and/or a lipid component in a prescribed weight ratio for a coating layer coating drug particles and the finding has now led to completion of the present invention.

That is, the present invention provides a particulate formulation containing drug particles and a first coating layer coating the drug particles, characterized in that the first coating layer contains a water-insoluble polymer, inorganic particles, and/or a lipid component and the lipid component contains a C₁₅ or higher fatty acid.
In the particulate formulation of the present invention, the water-insoluble polymer preferably contains an enteric polymer.
Further, the average particle diameter of the inorganic particles is preferably 1 to 1000 nm.
Further, the inorganic particles are preferably of silica and/or titanium dioxide.
Further, the inorganic particles are preferably subjected to hydrophobization.
Further, the inorganic particles are preferably contained in an amount of 0.5 to 2.4 parts by weight to 2.4 parts by weight of the water-insoluble polymer.
Further, the inorganic particles are preferably contained in an amount of 0. 5 to 2.4 parts by weight to 2.4 parts by weight of the drug particles.
Further, the lipid component preferably contains a C₁₅ to C₂₁ fatty acid and the C₁₅ to C₂₁ fatty acid is preferably stearic acid.
Further, the lipid component is preferably contained in an amount of 0.1 to 0.8 parts by weight to 2.4 parts by weight of the water-insoluble polymer.
Further, the particulate formulation of the present invention preferably further contains a second coating layer coating the first coating layer.
The first coating layer preferably contains a cellulose enteric polymer and the second coating layer preferably contains a water-insoluble polymer different from the cellulose enteric polymer.
The cellulose enteric polymer preferably contains hydroxypropylmethyl cellulose phthalate (HPMCP) and/or hydroxypropylmethyl cellulose acetate succinate (HPMCAS).
The water-insoluble polymer different from the cellulose enteric polymer contains at least one kind selected from the group consisting of ethyl cellulose, a methacrylic acid-methyl acrylate copolymer, a methyl methacrylate-butyl methacrylate-dimethylaminoethyl methacrylate copolymer, polyvinyl acetal diethylaminoacetate, an ethyl acrylate-methyl methacrylate-chlorotrimethylammonium ethyl methacrylate copolymer, and an ethyl acrylate-methyl methacrylate copolymer.
The particulate formulation of the present invention preferably has an average particle diameter of 0.1 to 200 µm.

The present inventors made various investigations and have consequently found that a particulate formulation with highly controlled dissolution of a drug can be simply produced by a spray drying method by forming a first coating layer on drug particles by a spray drying method and thereafter, further forming a second coating layer by a spray drying method and using different coating polymers for the first coating layer and the second coating layer, and the finding has now led to completion of a method for producing the particulate formulation of the present invention.

That is, the present invention provides a method for producing a particulate formulation, including the steps of: forming a first coating layer on drug particles by dissolving and/or suspending the drug particles in a first coating solution containing a cellulose enteric polymer and carrying out a spray drying method; and forming a second coating layer by suspending the drug particles coated with the first coating layer in a second coating solution containing a water-insoluble polymer different from the cellulose enteric polymer and carrying out a spray drying method.
In the method for producing a particulate formulation of the present invention, the first coating solution preferably contains a first polar solvent and the first polar solvent is preferably acetone.
The second coating solution preferably contains a second polar solvent different from the first polar solvent and the second polar solvent is preferably ethanol.
Hereinafter, the present invention will be described in detail.

The present invention provides a particulate formulation containing drug particles and a first coating layer coating the drug particles.
The particulate formulation of the present invention with such a configuration is characterized in that dissolution of the drug can be effectively controlled even in the case the average particle diameter is small.
The drug in the particulate formulation is not particularly limited as long as they are an active component which is physiologically active or pharmacologically active and examples thereof include polypeptides and nucleic acids having physiological activity, antipyretic, pain-relieving, and anti-inflammatory drugs, gout and hyperuricemia-curing drugs, hypnotic and sedative drugs, sleep inducing drugs, anti-anxiety drugs, antiepileptics, antipsychotic drugs, antidepressants, antiperkinsonian drugs, agonists for autonomic nervous system, cerebral ameliorators, allergy treatment drugs, antiallergic drugs, antianginal drugs, β-blockers, calcium antagonists, cardiotonics, antiarrhythmic drugs, diuretics, depressors, vasoconstrictors, vasodilators, agents for hyperlipidemia, hypertensors, bronchodilator-antiasthmatics, antitussive drugs, expectorants, peptic ulcer agents, digestants, cathartics, intestinal drugs, gastric antacids, antidiabetic drugs, hormone preparations, vitamin preparations, antibiotics, drugs for treating osteoporosis, antibacterial drugs, chemotherpeutics, antiviral drugs, antitumor drugs, muscle-relaxant drugs, anticoagulants, hemostatics, antituberculous agents, narcotic antagonists, bone resorption inhibitors, angiogenic inhibitors, agents affecting central nervous system, local anesthetics, antidizzing drugs, cardiovascular drugs, respiratory stimulants, antidiarrheals, cholagogues, agents affecting digestive organs, agents affecting urinary organs, agents for liver disease, etc. The above-mentioned drugs may be used alone or in combination of two or more kinds.

The drug particles can be produced by a conventionally known milling technique (a hammer mill, a sample mill, a pin mill, a jet mill, a bead mill, etc.).
Further, usable as the drug particles are, for example, drug-containing particles obtained by mixing a physiologically or pharmacologically acceptable formulation agent and drug-containing particles obtained by coating the surface of nuclear particles of a proper material with a drug by using a fluidized bed granulating apparatus, a stirring fluidized bed apparatus, a rotating fluidized bed apparatus, a centrifugal rotating apparatus, or a spray drying apparatus.

In the particulate formulation of the present invention, the drug particles preferably have an average particle diameter of 0.08 to 160 µm. If the average particle diameter is smaller than 0.08 µm, the surface area of the particulate formulation of the present invention becomes large and dissolution control of the drug sometimes becomes difficult. On the other hand, if the average particle diameter exceeds 160 µm, the formulation may possibly give a rough feeling or a foreign body sensation in the mouth when the particulate formulation of the present invention is taken. The average particle diameter of the drug particles is more preferably in the range of 0.4 to 80 µm and further preferably in the range of 0.8 to 32 µm.
The average particle diameter of the drug particles means same as the average particle diameter of the particulate formulation described later.

The particulate formulation of the present invention has a configuration that the drug particles are coated with a first coating layer.
The first coating layer contains a water-insoluble polymer, inorganic particles, and/or a lipid component.
In the following description, components to be used for the particulate formulation of the present invention together with the drug particles may be called collectively also as formulation components.
Various physiologically or pharmacologically acceptable materials can be used as the water-insoluble polymer. Specific examples of the water-insoluble polymer include sustained-release polymers, gastric polymers, enteric polymers, etc.

Examples of the sustained-release polymers include water-insoluble cellulose derivatives such as ethyl cellulose; water-insoluble acrylic acid copolymers such as an ethyl acrylate-methyl methacrylate-chlorotrimethylammonium ethyl methacrylate copolymer (e.g., Eudragit RS and Eudragit RL, both are from Evonik Industries), an ethyl acrylate-methyl methacrylate copolymer (e.g., Eudragit NE from Evonik Industries), etc.
Examples of the gastric polymers include polyvinyl derivatives such as polyvinyl acetal diethylaminoacetate; (meth)acrylic acid copolymers such as a methyl methacrylate-butyl methacrylate-dimethylaminoethyl methacrylate copolymer (e.g., Eudragit E from Evonik Industries), etc.
Examples of the enteric polymers include cellulose derivatives such as hydroxypropylmethyl cellulose acetate succinate, hydroxypropylmethyl cellulose phthalate, hydroxymethylethyl cellulose phthalate, carboxymethylethyl cellulose, cellulose acetate propionate, cellulose acetate phthalate, cellulose acetate succinate, cellulose acetate trimellitate, and methyl cellulose phthalate; (meth)acrylic acid copolymers such as methacrylic acid copolymer L (e.g., Eudragit L from Evonik Industries), methacrylic acid copolymer LD (e. g. , Eudragit LD from Evonik Industries), methacrylic acid copolymer S (e.g., Eudragit S from Evonik Industries), etc.
These water-insoluble polymers may be used alone or in combination of two or more kinds. Preferable examples are water-insoluble cellulose derivatives, water-insoluble acrylic acid copolymers, gastric polymers, enteric polymers, etc. and they may be used alone or in combination of two or more kinds.
The "(meth)acrylic" means acrylic or methacrylic.

In the particulate formulation of the present invention, the water-insoluble polymers more preferably contain an enteric polymer. It is made possible that the drug can be more effectively dissolved particularly in the intestines by making the first coating layer contain an enteric polymer and inorganic particles described later.
The "enteric polymer" means a polymer which is insoluble in an aqueous solution at a pH of less than 5 but is soluble in an aqueous solution at a pH value in the range of pH 5 to 7.
Examples of the enteric polymer include those mentioned above and above all, hydroxypropylmethyl cellulose phthalate and a (meth)acrylic acid copolymer are preferably used. Commercially available examples as these enteric polymers are HP 55 (product name, from Shin-Etsu Chemical Co., Ltd.) and Eudragit S100 (from Evonik Industries).

Various physiologically or pharmacologically acceptable materials can be used as the inorganic particles composing the first coating layer.
Examples of such inorganic particles include silica (hydrated silicon dioxide, lightweight silicic anhydride), titanium dioxide, talc, kaolin, magnesium stearate, calcium stearate, sodium stearyl fumarate, calcium carbonate, calcium phosphate, calcium silicate, magnesium silicate, aluminum silicate, magnesium silicate aluminate, magnesium metasilicate aluminate, magnesium aluminum silicate, lightweight aluminum oxide, etc. These inorganic particles may be used alone or in combination of two or more kinds.
These inorganic particles may be produced by a conventionally known milling and dispersing technique (a hammer mill, a sample mill, a pin mill, a jet mill, a bead mill, a high pressure homogenizer, ultrasonic milling and dispersing, etc.).

The inorganic particles are preferably those subjected to hydrophobization. Dissolution of the drug is made more highly controllable by subjecting the inorganic particles to hydrophobization.
Herein, "being subjected to hydrophobization" means chemical alkylation of the surface.
Specific examples of the hydrophobization include those achieved by covering the surface of lightweight silicic anhydride with methyl groups in the case of lightweight silicic acid subjected to hydrophobization and also those achieved by chemically treating titanium oxide with octylsilane in the case of titanium oxide subjected to hydrophobization.

The inorganic particles are especially preferably silica and/or titanium dioxide since commercialized products of them with an average particle diameter within the range described later are made available. Particularly, lightweight silicic anhydride subjected to hydrophobization (e.g., Aerosil-R972 from Nippon Aerosil Co., Ltd.) and titanium oxide subjected to hydrophobization (e.g., Aeroxide-T805 from Nippon Aerosil Co., Ltd.)are preferable.

The average particle diameter of the inorganic particles is preferably 1 to 1000 nm and a more preferable lower limit is 100 nm and a more preferable upper limit is 500 nm. If it is smaller than 1 nm, the handling property during the work may be worsened and those which can be purchased as a pharmaceutical additive are possibly not made available. On the other hand, if the average particle diameter exceeds 1000 nm, in the case of using such particles for the particulate formulation of the prevent invention, it becomes difficult to evenly and densely fill the first coating layer with the particles and thus control of drug dissolution may possibly become impossible.
Herein, the average particle diameter of the inorganic particles means same as the average particle diameter of the particulate formulation described later.

In the particulate formulation of the present invention, the content of the inorganic particles to that of the above-mentioned water-insoluble polymer is preferably controlled to be in the range of 0.5 to 2. 4 parts by weight and more preferably in the range of 1.0 to 2.0 parts by weight to 2.4 parts by weight of the water-insoluble polymer according to the type of drug particles, the desired degree of the dissolution control of the drug, etc. If the content of the inorganic particles to that of the water-insoluble polymer is less than 0.5 parts by weight, it may possibly become impossible to cause the effect of dissolution control of the drug. On the other hand, if the content exceeds 2.4 parts by weight, it may possibly cause fragility of the first coating layer.

The content of the inorganic particles to that of the above-mentioned drug particles is also preferably controlled in accordance with the type of the drug particles, the desired degree of the dissolution control, etc. and it is preferably 0.5 to 2.4 parts by weight and more preferably 1.0 to 2.0 parts by weight to 2.4 parts by weight of the drug particles. If the content of the inorganic particles to that of the drug particles is less than 0.5 parts by weight, it may possibly become impossible to cause the sufficient effect of dissolution control of the drug. On the other hand, if the content exceeds 2.4 parts by weight, it may possibly cause fragility of the first coating layer.

Various physiologically or pharmacologically acceptable materials can be used as the lipid component composing the first coating layer.
Examples of such lipid component include fatty acids (palmitic acid, stearic acid, arachidic acid, behenic acid, etc.), higher alcohols (cetyl alcohol, palmityl alcohol, stearyl alcohol, etc.), fatty acid esters (glyceryl monostearate, glyceryl monopalmitate, glyceryl behenate, etc.), hydrogenated oils (hydrogenated castor oil, etc.), solid fats and oils (cacao butter, etc.), and waxes (bees wax, white bees wax, carnauba wax, etc.). These lipid components may be used alone or in combination of two or more kinds.

In the particulate formulation of the present invention, the lipid component includes a C₁₅ or higher fatty acid. If the lipid component is a fatty acid of lower than C₁₅, it may possibly be impossible to sufficiently control dissolution of the drug.
The lipid component particularly preferably contains a C₁₅ to C₂₁ fatty acid. The dissolution of the drug can be effectively controlled by containing a C₁₅ to C₂₁ fatty acid as the lipid component, even in the case the average particle diameter of the particulate formulation of the present invention is small.
The C₁₅ or higher fatty acid means a fatty acid having 15 or more carbon atoms in total in the molecule.
The C₁₅ to C₂₁ fatty acid means a fatty acid having 15 to 21 carbon atoms in total in the molecule and, for example, palmitic acid, stearic acid, and arachidic acid are preferable and stearic acid is particularly preferable. The dissolution of the drug can be highly controlled by adding stearic acid as the lipid component.

In the particulate formulation of the present invention, the content of the lipid component to that of the above-mentioned water-insoluble polymer is controlled to be in the range of 0.1 to 0.8 parts by weight to 2.4 parts by weight of the water-insoluble polymer according to the type of drug particles, the desired degree of the dissolution control of the drug, etc. The content is preferably 0.15 to 0.75 parts by weight and more preferably 0.2 to 0.55 parts by weight to 2.4 parts by weight of the water-insoluble polymers.
If the content of the lipid component to that of the water-insoluble polymer is less than 0.1 parts by weight, it may possibly become difficult to dissolve the drug in a neutral region in the case the particulate formulation of the present invention is an enteric formulation and thus the characteristic as the enteric formulation cannot be exhibited sufficiently. On the other hand, if the content exceeds 0.8 parts by weight, it becomes difficult to form an effective first coating layer and it results in impossibility of dissolution control of the drug.

In addition, the first coating layer may contain, as necessary, conventionally known components to be used for pharmaceutical formulations, for example, water-soluble polymers, binders, disintegrants, waxes, plasticizers, saccharides, acidulants, artificial sweeteners, perfumes, lubricants, colorants, stabilizers, etc. as additives. These additives may be used alone or in combination of two or more kinds.

The first coating layer may contain surfactants, as necessary, in order to improve the dispersibility of the drug particles in a coating solution described later. These surfactants may be used alone or in combination of two or more kinds. Examples of the surfactant include glyceryl monoisostearate and the like.

The first coating layer can be formed by using, as a coating solution, for example, a solution (an organic solvent solution, etc.) or a dispersion (a water dispersion, etc.) containing the above-mentioned water-insoluble polymer, inorganic particles, and/or lipid component as well as other additives (formulating components).
Examples of the solvent in the coating solution include water and organic solvents. Examples of the organic solvents include alcohols (ethanol, propanol, isopropanol, etc.), hydrocarbons (n-hexane, cyclohexane, etc.), halogenated alkanes (dichloromethane, chloroform, trichloroethane, carbon tetrachloride, etc.), ketones (acetone, methyl ethyl ketone, etc.), esters (ethyl acetate, etc.), ethers, nitriles (acetonitrile, etc.), etc.
These organic solvents may be used in combination of two or more kinds and water-compatible organic solvents may be used as mixed solvents with water.
Alcohols, ketones, and water are preferable among these solvents.

The concentration of the water-insoluble polymer in the coating solution may be selected in accordance with the viscosity and spraying property of the coating solution and it is, for example, 0.1 to 50 weight %, preferably 0.2 to 40 weight %, more preferably about 0.5 to 25 weight %, and even more preferably about 1 to 10 weight %. The coating solution may contain the above-mentioned various kinds of additives.

The first coating layer may be formed on the surface of the drug particles by a conventionally known coating technique using, for example, a stirring fluidized bed apparatus, a rotating fluidized bed apparatus, a Wurster fluidized bed apparatus, or a spray drying apparatus. Especially, for production of the particulate formulation of the present invention, a spray drying method using a spray drying apparatus is suitable.

In the spray drying method, first, a coating solution is prepared by mixing or suspending drug particles and the respective materials composing the first coating layer and the coating solution is sprayed to a drying chamber of a spray dryer using a nozzle. Water or an organic solvent in the finely granulated droplets formed by the spraying is evaporated within an extremely short time so that the particulate formulation containing drug particles coated with the first coating layer can be produced.
Examples of the type of nozzle include a two-fluid nozzle type, a multi-fluid nozzle type, a pressure nozzle type, a rotary disk type, etc.
The nozzle type and spraying conditions are not particularly limited as long as they are suitable for spraying the coating solution prepared as described above. The conditions may be properly selected in accordance with the composition of the particulate formulation and the spray dryer to be used. For example, in the case of a two-fluid nozzle type spray dryer, the air intake temperature is properly adjusted in accordance with the solvent to be used and generally, it is about 30 to 200°C and preferably about 40 to 150°C. The amount of the spraying solution is properly adjusted in accordance with the scale of the apparatus and it is generally about 1 to 30 mL/min in the case of, for example, a laboratory scale (chamber diameter of 15.5 cm). The flow rate of the sprayed gas is adjusted generally in the range of about 50 to 900 L/hr, although it depends on the particle diameter of the particulate formulation to be produced and nozzle diameter.
In the particulate formulation, if necessary, water or the solvent in the drug particles may be completely removed under reduced pressure.

The average particle diameter of the particulate formulation of the present invention is not particularly limited as long as it is within a range in which the rough feeling in the mouth can be lessened and it is preferably 0.1 to 200 µm, more preferably 0.5 to 100 µm, furthermore preferably 1.0 to 40 µm, and most preferably 1.0 to 15 µm. If the average particle diameter is smaller than 0.1 µm, the surface area of the particulate formulation of the present invention becomes large and dissolution control of the drug sometimes becomes difficult. On the other hand, if the average particle diameter exceeds 200 µm, it may possibly give a rough feeling or a foreign body sensation in the mouth.
Herein, the "average particle diameter" means the particle diameter of 50% by volume of the entire particles and may be measured by using an "automatic particle image analyzer" (a particle image analyzer is an apparatus for measuring the particle diameter from images of photographed particles) and a "laser diffraction particle size distribution analyzer" (or a "laser diffraction/scattering particle size distribution analyzer").

Further, the particulate formulation of the present invention preferably further contains a second coating layer coating the first coating layer. Dissolution of the drug can be made more highly controllable by coating the drug particles with the first coating layer and the second coating layer.
In the particulate formulation of the present invention having the first coating layer and the second coating layer, it is preferable that the first coating layer contains a cellulose enteric polymer and the second coating layer contains a water-insoluble polymer different from the cellulose enteric polymer.

The particulate formulation of the present invention having the first coating layer and the second coating layer can be produced by a method including the steps of forming a first coating layer on drug particles by dissolving and/or suspending the drug particles in a first coating solution containing a cellulose enteric polymer and carrying out a spray drying method and forming a second coating layer by suspending the drug particles coated with the first coating layer in a second coating solution containing a water-insoluble polymer different from the cellulose enteric polymer and carrying out a spray drying method. The production method of the particulate formulation is also one of the present inventions.

In the step of forming the first coating layer, the drug particles are dissolved and/or suspended in a first coating solution containing a cellulose enteric polymer.
A method for dissolving and/or suspending the drug particles in the first coating solution is not particularly limited and can be carried out by a conventionally known method.

Various physiologically or pharmacologically acceptable materials can be used as the cellulose enteric polymer. The cellulose enteric polymer is especially preferably water-insoluble and examples of the cellulose enteric polymer include hydroxypropylmethyl cellulose acetate succinate (HPMCAS), hydroxypropylmethyl cellulose phthalate (HPMCP), hydroxymethylethyl cellulose phthalate, carboxymethylethyl cellulose, cellulose acetate propionate, cellulose acetate phthalate, cellulose acetate succinate, cellulose acetate trimellitate, methyl cellulose phthalate, etc.
These cellulose enteric polymers may be used alone or in combination of two or more kinds.
Especially, hydroxypropylmethyl cellulose acetate succinate (HPMCAS) and/or hydroxypropylmethyl cellulose phthalate (HPMCP) is preferably used as the cellulose enteric polymer. In the case of using these cellulose enteric polymers, it is made easy to form the first coating layer for effectively controlling dissolution.
In addition, the "enteric polymer" means a polymer which is insoluble in an aqueous solution at a pH of lower than 5 but is soluble in an aqueous solution at a pH value in the range of pH 5 to 7.

The first coating solution may contain the above-mentioned inorganic particles and also, as necessary, conventionally known components to be used for pharmaceutical formulations, for example, water-soluble polymers, binders, disintegrants, waxes, plasticizers, saccharides, acidulants, artificial sweeteners, perfumes, lubricants, colorants, stabilizers, etc., as additives. These additives may be used alone or in combination of two or more kinds.

A solution (an organic solvent solution), a dispersion (a water dispersion), or the like containing the above-mentioned cellulose enteric polymer, inorganic particles, and other additives (formulating components) can be used as the first coating solution.
Examples of the solvent include water and organic solvents.
Examples of the organic solvents include alcohols (ethanol, propanol, isopropanol, etc.), hydrocarbons (n-hexane, cyclohexane, etc.), halogenated alkanes (dichloromethane, chloroform, trichloroethane, tetrachloromethane, etc.), ketones (acetone, methyl ethyl ketone, etc.), esters (ethyl acetate, etc.), ethers, nitriles (acetonitrile, etc.), etc. These organic solvents may be used in combination of two or more kinds and water-compatible organic solvents may be used as mixed solvents with water.
As the organic solvent, a polar organic solvent (hereinafter, referred to as a first polar solvent) is preferable, and more specifically, preferable examples include ketones (e. g. , acetone), mixed solvents of ketones and alcohols (e.g., ethanol, methanol), and mixed solvents of ketones and halogenated alkanes (e.g., dichloromethane). Among them, acetone is particularly preferable as the first polar solvent.

The concentration of the cellulose enteric polymer in the first coating solution is selected in accordance with the viscosity and spraying property of the first coating solution and it is, for example, preferably 0.1 to 50% by weight, more preferably 0.2 to 40% by weight, furthermore preferably about 0. 5 to 25% by weight, and most preferably about 1 to 10% by weight. If the concentration of the cellulose enteric polymer is less than 0.1% by weight, it may possibly become impossible to produce the particulate formulation with sufficient dissolution control of the drug. On the other hand, if the concentration exceeds 50% by weight, the first coating solution becomes highly viscous and it may sometimes result in impossibility of forming a uniform first coating layer.

In the spray drying method, first, the first coating solution prepared by dissolving or suspending drug particles by the above-mentioned method is sprayed to a drying chamber of a spray dryer using a nozzle. Water or the organic solvent in the finely granulated droplets is evaporated within an extremely short time so that the first coating layer can be formed on the drug particles.
Examples of the nozzle include a two-fluid nozzle type, a multi-fluid nozzle type, a pressure nozzle type, a rotary disk type, etc.
The nozzle type and spraying conditions are not particularly limited as long as they are suitable for spraying the first coating solution prepared by dissolving and/or suspending the drug particles as described above. The conditions may be properly selected in accordance with the composition of the particulate formulation to be produced and the spray dryer to be used. For example, in the case of a two-fluid nozzle type spray dryer, the air intake temperature is properly adjusted in accordance with the solvent to be used and generally, it is about 50 to 200°C and preferably about 80 to 150°C. The amount of the spraying solution is properly adjusted in accordance with the scale of the apparatus and it is generally about 1 to 30 mL/min in the case of, for example, a laboratory scale (chamber diameter of 15.5 cm). The flow rate of the sprayed gas is adjusted generally in the range of about 50 to 900 L/hr, although it depends on the particle diameter of the particulate formulation to be produced and nozzle diameter.
In this step, if necessary, water or the solvent in the drug particles may be completely removed under reduced pressure.

The average particle diameter of the particles coated with the first coating layer and obtained in this step is preferably 0.09 to 180 µm, more preferably 0.45 to 90 µm and even more preferably 0.9 to 36 µm. If the average particle diameter is smaller than 0.09 µm, dissolution control of the drug sometimes becomes difficult in the particulate formulation to be produced according to the present invention. On the other hand, if the average particle diameter exceeds 180 µm, the particulate formulation produced according to the present invention may possibly give a rough feeling or a foreign body sensation in the mouth when the particulate formulation is taken.

The method for producing a particulate formulation of the present invention, then, has a step of forming a second coating layer by suspending the drug particles coated with the first coating layer in a second coating solution containing a water-insoluble polymer different from the cellulose enteric polymer and carrying out a spray drying method.
The second coating layer can be formed on the drug particles coated with the first coating layer by a spray drying method in this step to produce the intended particulate formulation.

In this step, first, the drug particles coated with the first coating layer are suspended in a second coating solution.
The second coating solution is a solution containing a water-insoluble polymer different from the cellulose enteric polymer. As described later, examples of the water-insoluble polymer contained in the second coating solution include the same polymers as the above-mentioned cellulose enteric polymers; however, in the present invention, different polymers from the above-mentioned cellulose enteric polymers are used as the water-insoluble polymer to be used in this step. In the second coating solution, it is only required that the type of the cellulose enteric polymer is different from that of the water-insoluble polymer.
Use of the second coating solution containing such a water-insoluble polymer can give the particulate formulation to be produced by the present invention a structure of multilayer coating on the drug particles and thus can highly control dissolution of the drug.

Various physiologically or pharmacologically acceptable materials can be used as the water-insoluble polymer. Examples of the water-insoluble polymer include sustained-release polymers, gastric polymers, enteric polymers, etc.

Examples of the sustained-release polymers include water-insoluble cellulose derivatives such as ethyl cellulose; water-insoluble acrylic acid copolymers such as an ethyl acrylate-methyl methacrylate-chlorotrimethylammonium ethyl methacrylate copolymer (e.g., Eudragit RS and Eudragit RL, both are from Evonik Industries), and an ethyl acrylate-methyl methacrylate copolymer (e.g., Eudragit NE from Evonik Industries), etc.
Examples of the gastric polymers include polyvinyl derivatives such as polyvinyl acetal diethylaminoacetate; (meth)acrylic acid copolymers such as a methyl methacrylate-butyl methacrylate-dimethylaminoethyl methacrylate copolymer (e.g., Eudragit E from Evonik Industries), etc.
Examples of the enteric polymers include (meth)acrylic acid copolymers such as methacrylic acid copolymer L (e.g., Eudragit L from Evonik Industries), methacrylic acid copolymer LD (e.g., Eudragit LD from Evonik Industries), methacrylic acid copolymer S (e.g., Eudragit S from Evonik Industries), etc.
These water-insoluble polymers may be used alone or in combination of two or more kinds. Preferable examples are water-insoluble cellulose derivatives, gastric polymers, enteric polymers, etc. and they may be used alone or in combination of two or more kinds.
The "(meth)acrylic" means acrylic or methacrylic.

Among them, preferable as the water-insoluble polymer are ethyl cellulose, methacrylic acid copolymer S, polyvinyl acetal diethylaminoacetate, and a methyl methacrylate-butyl methacrylate-dimethylaminoethyl methacrylate copolymer. Particularly, ethyl cellulose is preferable. In the case ethyl cellulose is used as the water-insoluble polymer, the second coating layer for effectively controlling dissolution can be formed easily.

The second coating solution may contain inorganic particles same as those in the above-mentioned first coating solution.
The content of the inorganic particles is properly determined in accordance with the type of the drug of the above-mentioned drug particles, the desired degree of the dissolution control, etc. The content is preferably 0.5 to 2.0 parts by weight and more preferably 1.0 to 2.0 parts by weight to 2.4 parts by weight of the water-insoluble polymer. If the content of the inorganic particles is less than 0.5 parts by weight, it may possibly become impossible to cause the effect of dissolution control. On the other hand, if the content exceeds 2.0 parts by weight, it may possibly cause fragility of the second coating layer.

The second coating solution may_contain, as necessary, conventionally known components to be used for pharmaceutical formulations, for example, water-soluble polymers, binders, disintegrants, waxes, plasticizers, saccharides, acidulants, artificial sweeteners, perfumes, lubricants, colorants, stabilizers, etc., as additives. These additives may be used alone or in combination of two or more kinds.

The second coating solution preferably contains the above-mentioned water-insoluble polymers, inorganic particles, and other additives (formulating components) as well as a second polar solvent different from the above-mentioned first polar solvent.
The second polar solvent is different from the first polar solvent. Particularly, the second polar solvent is different from the first polar solvent in the dissolution property. More specifically, the second polar solvent is different from the first polar solvent in that the second polar solvent does not substantially dissolve the enteric cellulose polymer to be used in the first coating layer. In addition, "do not substantially dissolve the enteric cellulose polymer" means that the weight of the enteric cellulose polymer soluble in 100 g of the second polar solvent is 1 g or less (under the condition of room temperature 25°C).
The particulate formulation coated with bilayer coating can be produced according to the present invention and the particulate formulation is provided with highly controlled dissolution by using the second polar solvent different from the first polar solvent. In the case the above-mentioned water-soluble drug is used, since a polar organic solvent does not cause any effect on the drug having water solubility, the second polar solvent is preferably a polar organic solvent.

Examples of the second polar solvent include alcohols (e.g., ethanol and methanol) and halogenated alkanes (e.g., dichloromethane) and especially, ethanol is preferably used.
The concentration of the water-insoluble polymer in the second coating solution is selected in accordance with the viscosity and spraying property of the second coating solution and it is, for example, 0.1 to 50 weight %, preferably 0.2 to 40 weight %, more preferably about 0.5 to 25 weight %, and most preferably about 1 to 10 weight %. If the concentration of the water-insoluble polymer is less than 0.1% by weight, it may possibly become impossible to produce the particulate formulation with sufficient dissolution control of the drug. On the other hand, if the concentration exceeds 50% by weight, the second coating solution becomes highly viscous and it may sometimes result in impossibility of forming a uniform second coating layer.

A spray drying method using the second coating solution may be the same method as the above-mentioned spray drying method using the first coating solution.

According to the method for producing the particulate formulation of the present invention including the above-mentioned steps, a bilayer-coated particulate formulation containing drug particles coated with the above-mentioned first and second coating layers can be produced and the particulate formulation with such a structure is provided with highly controlled dissolution of the drug.
Further, in the steps of forming the first coating layer and the second coating layer, there is no need to employ a complicated method of simultaneously spraying two types of spraying solutions from different nozzles and the particulate formulation with the above-mentioned configuration can be simply produced by the spray drying method.

The average particle diameter of the particulate formulation coated with the second coating layer is not particularly limited as long as it is within a range in which the rough feeling in the mouth can be lessened and it is preferably 0.1 to 200 µm, more preferably 0.5 to 100 µm, furthermore preferably 1.0 to 50 µm, and most preferably 1.0 to 40 µm.
If the average particle diameter of the particulate formulation is smaller than 0.1 µm, the surface area of the particulate formulation becomes large and dissolution control sometimes becomes difficult. On the other hand, if the average particle diameter of the particulate formulation exceeds 200 µm, the formulation may possibly give a rough feeling or a foreign body sensation in the mouth. Herein, the average particle diameter of the particulate formulation is a value measured in the same manner as in the average particle diameter of the particulate formulation of the present invention described above.

The particulate formulation of the present invention can be administered as it is in form of a fine granule to a living body and also can be administered after being formed into various kinds of formulations and can thus be used as a starting substance for producing such formulations.
Examples of the formulations include injectables, formulations for oral administration (e.g., powdered drugs, granules, capsules, tablets, orally disintegrating tablets, dry-syrups, suspensions/emulsions, and film agents), drugs for transnasal administration, suppositories (e.g., rectal suppositories and vaginal suppositories), etc.
The amount of the drug particles in these formulations may vary in accordance with the type of drug, the administration form, the object disease, etc. and in general, it is preferably 0.001 mg to 5 g and more preferably about 0.01 mg to 2 g per one formulation.

The formulation can be produced by a generally employed and conventionally known method.
Specifically, the particulate formulation of the present invention may be formed into an injectable by forming the particulate formulation in the form of an aqueous suspension using a dispersant (e.g., Tween 80 from Atlas Powder, USA), HCO 60 (from Nikko Chemicals Co., Ltd.), carboxymethyl cellulose, sodium alginate, etc.), a preservative (e.g., methylparaben, propylparaben, benzyl alcohol, chlorobutanol, etc.), a tonicity agent (e.g., sodium chloride, glycerin, sorbitol, glucose, etc.) etc., or by dispersing the particulate formulation in a plant oil such as olive oil, sesame oil, peanut oil, cottonseed oil, or corn oil, or propylene glycol to form into an oil suspension.

The formulations for oral administration may be produced according to a conventionally known method using the particulate formulation of the present invention and a commonly used formulation agent.
Examples of the formulation agent include commonly used components of the formulations for oral administration, such as water-soluble polymers or binders (e.g., hydroxypropylmethyl cellulose, hydroxypropyl cellulose, methyl cellulose, ethyl cellulose, hydroxyethyl cellulose, carboxymethyl cellulose, crystalline cellulose/sodium carboxymethyl cellulose, polyvinylpyrrolidone, polyvinyl alcohol, gum arabic powder, gelatin, pullulan, etc.), excipients (crystalline cellulose, starch such as corn starch, saccharides such as sucrose, lactose, powdered sugar, granulated sugar, glucose, and mannitol, lightweight silicic anhydride, talc, magnesium carbonate, calcium carbonate, etc.), disintegrants (e.g., starch such as corn starch, calcium carmellose, crospovidone, hydroxypropyl cellulose with a low substitution degree, etc.), lipids (e.g., hydrocarbons, waxes, higher fatty acids and their salts, higher alcohols, fatty acid esters, hydrogenated oils such as hydrogenated castor oil), plasticizers (triacetin, triethyl citrate, dibutyl sebacate, etc.), corrigents (e.g., sweeteners (sugars such as sucrose, lactose, glucose, and maltose, sugar alcohols such as mannitol, xylitol, sorbitol, and erythritol, artificial sweeteners such as saccharin, sodium saccharin, aspartame, and stevia); acidulants such as citric acid, tartaric acid, and malic acid; perfumes such as lemon, lemonlime, orange, and menthol), lubricants (e.g., magnesium stearate, calcium stearate, stearic acid, talc, etc.), fluidizing agents (e.g., lightweight silicic anhydride), colorants (e.g., food dyes, caramel, red iron oxide, titanium oxide, etc.), surfactants (e.g., anionic surfactants such as sodium alkylsulfate, nonionic surfactants such as polyoxyethylene sorbitan fatty acid esters, polyoxyethylene fatty acid esters, and polyoxyethylene castor oil derivatives, etc.), wetting agents (polyethylene glycol (macrogol), etc.) fillers, extenders, adsorbents, preservers such as antiseptics or stabilizers, antistatic agents, disintegration-extending agents, etc.
Further, the formulations for oral administration may be coated, as necessary, by a conventionally known method for masking the taste, solubility, enteric solubility, or sustainability.

Further, in the case of obtaining, for example, a drug for transnasal administration, the particulate formulation of the present invention may be formed into a solid, semi-solid, or liquid drug for transnasal administration by a conventionally known method. For example, as the solid drug, the particulate formulation of the present invention may be used as it is or an excipient (e.g., glucose, mannitol, starch, microcrystalline cellulose, etc.), a thickener (e.g., natural gums, cellulose derivatives, acrylic acid polymers, etc.) etc. are added and mixed to obtain a powder compositions.

In the case of the liquid formulation, almost same as the case of an injectable, the particulate formulation is made into an oil or aqueous suspension. In the case of a semi-solid type, an ointment is preferable. These formulations may contain a pH adjusting agent (e.g., carbonic acid, phosphoric acid, citric acid, hydrochloric acid, sodium hydroxide, etc.), an antiseptic (e.g. p-oxybenzoic acid esters, chlorobutanol, benzalkonium chloride, etc.) etc.

Further, in the case of obtaining, for example, a suppository, the particulate formulation of the present invention may be formed in an oil- or water-based solid, semi-solid, or liquid suppository by adding an oleaginous base or aqueous base according to a conventionally known method.
The oleaginous base may be one which does not dissolve the particulate formulation and examples thereof include higher fatty acid glycerides (e.g., cacao butter, witepsol, etc.), middle fatty acids (e.g., miglitols, etc.), plant oils (e.g., sesame oil, soybean oil, cottonseed oil, etc.) etc. Examples of the aqueous base include polyethylene glycol, propylene glycol, etc. In the case of obtaining the suppository, as an aqueous gel base, natural gums, cellulose derivatives, vinyl polymers, acrylic acid polymers, etc. may be used.

The formulation for oral administration may be obtained by a commonly used method in accordance with the dosage form, for example, by granulating or kneading the particulate formulation of the present invention as necessary and formulating the obtained material.
The granulation of the particulate formulation of the present invention may be carried out by a commonly used granulation method, for example, by a rotating granulation method or a fluidized bed granulation method, and granulation and kneading are carried out generally using a carrier such as an excipient or a binder. In the case of, for example, a tablet, the particulate formulation of the present invention may be mixed or kneaded with a carrier such as an excipient or a binder and granulated as necessary, mixed with an additive such as a lubricant, and compacted into a tablet. In the case of, for example, a capsule, the particulate formulation of the present invention or a granule thereof may be encapsulated to obtain a capsule.

### - Advantageous Effects of Invention

Since the particulate formulation of the present invention has a coating layer coating drug particles, which contains a water-insoluble polymer, inorganic particles, and/or a lipid component in a prescribed weight ratio, the drug particles can be effectively coated with the first coating layer and dissolution of the drug can be effectively suppressed even in the case the diameter of the drug particles is small.
In a preferable embodiment in which the water-insoluble polymer contains an enteric polymer, since the first coating layer contains a specific lipid component, the drug can be more effectively dissolved particularly in the intestines.

### DESCRIPTION OF EMBODIMENTS

Hereinafter, the present invention will be concretely described with reference to examples; however, the present invention should not be limited to these examples.

The following drugs and formulation components were used in the following examples and comparative examples.

### (Drugs)

Potassium guaiacolsulfonate
Tamsulosin hydrochloride

### (Formulation components)

Ethyl cellulose (Ethocel 10 from Dow Chemical)
HPMCP;hydroxypropylmethyl cellulose phthalate (HP 55 from Shin-Etsu Chemical Co., Ltd.)
Eudragit S100 (Eudragit-S100 from Evonik Industries)
Myristic acid (special grade reagent from Wako Pure Chemical Industries Ltd.)
Palmitic acid (special grade reagent from Wako Pure Chemical Industries Ltd.)
Stearic acid (special grade reagent from Wako Pure Chemical Industries Ltd.)
Arachidic acid (special grade reagent from Wako Pure Chemical Industries Ltd.)
Behenic acid (special grade reagent from Wako Pure Chemical Industries Ltd.)
Hydrophobic lightweight silicic anhydride (Aerosil-R972 from Nippon Aerosil Co., Ltd.)
Titanium oxide (Aeroxide-P-25 and Aeroxide-T805 from Nippon Aerosil Co., Ltd.)
Glyceryl monoisostearate (NIKKOL-MGIS from Nikko Chemicals Co., Ltd.)

Particulate formulations were produced and evaluated in the following examples and comparative examples according to the following methods.

### (Production of particulate formulation containing potassium guaiacolsulfonate)

A solution was obtained by adding and dissolving 100 g of potassium guaiacolsulfonate in 900 g of water. The solution was spray-dried with a spray dryer (Mini Spray Dryer B-290 from BUCHI Labortechnik AG) at a spraying solution amount of 2 mL/min, an air intake temperature of 120°C, and a spraying air flow rate of 190 L/hr to obtain spherical potassium guaiacolsulfonate particles as drug particles.

### (Production of Tamsulosin hydrochloride particles)

Tamsulosin hydrochloride was physically milled by a jet mill (Spiral Jet Mill 50 AS from Hosokawa Micron Corporation) to obtain tamsulosin hydrochloride particles as drug particles.

### (Coating)

The formulation components and drug particles were added to solvents and suspended. The obtained suspensions were spray-dried by a spray dryer (Mini Spray Dryer B-290 from BUCHI Labortechnik AG) to obtain particulate formulations. The compositions of the formulation components used in respective examples and comparative examples are shown in the tables.

### (Measurement of average particle diameter)

### <Average particle diameter of particulate formulation>

The average particle diameter was measured using 20000 or more particles with an automatic particle image analyzer (Morphologi (registered trade name)-G3 from Malvern Instruments).

### <Average particle diameter of inorganic particles>

The average particle diameter of the inorganic particles was previously measured before production of particulate formulations.
That is, an ethanol suspension of inorganic particles was prepared and subjected to ultrasonic treatment and thereafter, the average particle diameter was measured with a particle diameter measurement system ELSZ-1 (from Otsuka Electronics, Co., Ltd., by a dynamic and electrophoretic light scattering method). The average particle diameters of the inorganic particles used in examples are shown below.
Aerosil-A200 (average particle diameter of 400.5 nm) as inorganic particles not subjected to hydrophobization; Aerosil-R972 (average particle diameter of 218.0 nm) as inorganic particles subjected to hydrophobization; Aeroxide-P25 (average particle diameter of 128.2 nm) as inorganic particles not subjected to hydrophobization; and Aeroxide-T805 (average particle diameter of 111.4 nm) as inorganic particles subjected to hydrophobization.

### (Dissolution test)

According to the flow-through cell method disclosed in the Japanese Pharmacopoeia, the test was carried out using a dissolution medium which was one of 1st fluid for dissolution test(pH 1.2), 2nd fluid for dissolution test (pH 6.8), and a disodium hydrogen phosphate-citric acid buffer solution at pH 7.2 (obtained by dissolving 7.1 g of dehydrated disodium hydrogen phosphate in water and adjusting the total volume to be 1000 mL, and adjusting the pH to 7.2 by adding, to the obtained solution, a solution obtained by dissolving 5.3 g of citric acid monohydrate in water and adjusting the total volume to be 1000 mL).
Swinnex Filter Holder 25 mm (from Nippon Millipore) was used for a cell. In order to prevent flowing out of the particulate formulations, a support screen (25 mm stainless, from Nippon Millipore), Durapore Membrane Filters (PVDF, Hydrophilic, 0.45 µm, 25 mm, from Nippon Millipore), and a silicon gasket 25 mm (Swinnex Filter Holder from Nippon Millipore) were successively installed. Masterflex L/S (Model 7524-50 from Cole Parmer Instrument COMPANY) was used as a pump and a dissolution medium was transported at 2. 5 mL/min. Besides that, the test was carried out according to the flow-through cell method disclosed in the Japanese Pharmacopeia. An eluate was sampled 10, 30, 60 minutes after starting the test and the dissolution amount of the drug in the eluate was measured by high performance liquid chromatography (HPLC) and the dissolution rate to the initial drug content was calculated.

### (Examples 1 to 5)

Ethyl cellulose as a sustained release polymer was used as the water-insoluble polymer, potassium guaiacolsulfonate drug particles were used as the drug particles, and the types of inorganic particles were changed to prepare particulate formulations by the above-mentioned method. The composition and results of various tests are shown in Table 1.

### (Comparative Example 1)

A particulate formulation was prepared in the same manner as in Example 1, except that no inorganic particles were added. The composition and results of various tests are shown in Table 1.

**[Table 1]**

| Composition | | Comparative Example 1 | | Example 1 | | Example 2 | | Example 3 | | Example 4 | | Example 5 | |
|---|---|---|---|---|---|---|---|---|---|---|---|---|---|
| Formulation components | Ethyl cellulose(g) | 2.4 | | 2.4 | | 2.4 | | 2.4 | | 2.4 | | 2.4 | |
| | Aerosil-A200(g) | - | | 0.9 | | 1.5 | | - | | - | | - | |
| | Aerosil-R972(g) | - | | - | | - | | 0.5 | | 0.9 | | 1.5 | |
| | Glyceryl monoisostearate(g) | 0.06 | | 0.06 | | 0.06 | | 0.06 | | 0.06 | | 0.06 | |
| | Pharmaceutical particles(g) | 2.4 | | 2.4 | | 2.4 | | 2.4 | | 2.4 | | 2.4 | |
| Solvent | Ethanol(g) | 47.4 | | 46.5 | | 45.9 | | 46.9 | | 46.5 | | 45.9 | |
| Inorganic particles wt%(to a water-insoluble polymer) | | 0 | | 37.5 | | 62.5 | | 20.8 | | 37.5 | | 62.5 | |
| Inorganic particles wt%(to pharmaceutical particles) | | 0 | | 37.5 | | 62.5 | | 20.8 | | 37.5 | | 62.5 | |
| Pharmaceutical elution rate (%) | | pH1.2 | pH6.8 | pH1.2 | pH6.8 | pH1.2 | pH6.8 | pH1.2 | pH6.8 | pH 1.2 | pH6.8 | pH1.2 | pH6.8 |
| | 10min | 21.0 | 20.9 | 14.5 | 14.3 | 15.0 | 15.1 | 14.0 | 14.1 | 13.3 | 13.1 | 9.9 | 9.6 |
| | 30min | 39.0 | 38.8 | 28.3 | 28.0 | 30.5 | 30.0 | 25.0 | 25.9 | 22.5 | 22.0 | 19.0 | 18.6 |
| | 60min | 60.0 | 59.5 | 39.0 | 38.7 | 40.0 | 40.5 | 39.0 | 38.3 | 35.0 | 34.8 | 30.5 | 30.3 |
| Average particle diameter (*µ*m) | | 10.6 | | 8.5 | | 13.2 | | 9.4 | | 10.3 | | 11.1 | |

| | | | | | | | | | | | | | |
|---|---|---|---|---|---|---|---|---|---|---|---|---|---|
| Spraying solution amount (5 mL/min), air intake temperature (120°C), and spraying air flow rate (470 L/hr) | | | | | | | | | | | | | |

As shown in Table 1, it was found that the particulate formulations of examples containing a sustained release polymer as a water-insoluble polymer and inorganic particles in a prescribed weight ratio could effectively control (suppress) the drug dissolution and showed the characteristic as a sustained release formulation.
On the other hand, the particulate formulation of Comparative Example 1 containing no inorganic particles failed to effectively control (suppress) the drug dissolution.

### (Examples 6 and 7)

HPMCP as an enteric polymer for a water-insoluble polymer, inorganic particles (Aerosil-R972), and potassium guaiacolsulfonate drug particles as the drug particles were used to prepare particulate formulations. The composition and results of various tests measured in the same manner as in Example 1 are shown in Table 2.

### (Comparative Example 2)

A particulate formulation was prepared in the same manner as in Example 6, except that no inorganic particles were added. The composition and results of various tests are shown in Table 2.

**[Table 2]**

| Compositions | | Comparative Example 2 | | Example 6 | | Example 7 | |
|---|---|---|---|---|---|---|---|
| Formulation components | HPMCP(g) | 2.40 | | 2.40 | | 2.40 | |
| | Aerosil-R972(g) | - | | 1.50 | | 1.80 | |
| | Glyceryl monoisostearat^{.}e(g) | 0.060 | | 0.060 | | 0.060 | |
| | Pharmaceutical particles(g) | 2.40 | | 2.40 | | 2.40 | |
| Solvent | Acetone(g) | 47.40 | | 45.90 | | 45.60 | |
| Inorganic particles wt%(to a water-insoluble polymer) | | 0.0 | | 62.5 | | 75.0 | |
| Inorganic particles wt%(to pharmaceutical particles) | | 0.0 | | 62.5 | | 75.0 | |
| Pharmaceutical elution rate (%) | | pH1.2 | pH6.8 | pH1.2 | pH6.8 | pH1.2 | pH6.8 |
| | 10min | 97.4 | 100 | 34.0 | 61.1 | 19.7 | 36.2 |
| | 30min | 99.3 | 100 | 61.0 | 90.6 | 33.8 | 59.8 |
| | 60min | 100 | 100 | 85 | 100 | 47.8 | 79.9 |
| Average particle diameter(*µ*m) | | 9.1 | | 8.9 | | 9.2 | |

| | | | | | | | |
|---|---|---|---|---|---|---|---|
| Spraying solution amount (5 mL/min), air intake temperature (80°C), and spraying air flow rate (470 L/hr) | | | | | | | |

As shown in Table 2, it was found that the particulate formulations of examples containing the enteric polymer as a water-insoluble polymer and inorganic particles in a prescribed weight ratio could effectively control (suppress) the drug dissolution under a condition of pH 1.2 and could promote drug dissolution under a condition of pH 6.8 and thus showed the characteristic as an enteric drug.
On the other hand, the particulate formulation of Comparative Example 2 containing no inorganic particles failed to effectively control (suppress) the drug dissolution.

### (Examples 8 to 17)

Ethyl cellulose as a sustained release polymer and Eudragit S100 as an enteric polymer were used for water-insoluble polymers, potassium guaiacolsulfonate drug particles were used as the drug particles, and the types of inorganic particles were changed to prepare particulate formulations. The composition and results of various tests measured in the same manner as in Example 1 are shown in Table 3.

### (Comparative Example 3)

A particulate formulation was prepared in the same manner as in Example 8, except that no inorganic particles were added. The composition and results of various tests are shown in Table 3.

**[Table 3]**

| Composition | | Comparative Example 3 | | Example 8 | | Example 9 | | Example 10 | | Example 11 | | Example 12 | | Example 13 | | Example 14 | | Example 15 | | Example 16 | | Example 17 | |
|---|---|---|---|---|---|---|---|---|---|---|---|---|---|---|---|---|---|---|---|---|---|---|---|
| Formulation components | Ethyl callulose(g) | 1.20 | | 1.20 | | 1.20 | | 1.20 | | 1.20 | | 1.20 | | 1.20 | | 1.20 | | 1.20 | | 1.20 | | 1.20 | |
| | Eudaragit-S100(g) | 1.20 | | 1.20 | | 1.20 | | 1.20 | | 1.20 | | 1.20 | | 1.20 | | 1.20 | | 1.20 | | 1.20 | | 1.20 | |
| | Aerosil-A200(g) | - | | 1.20 | | 1.50 | | - | | - | | - | | - | | - | | - | | - | | - | |
| | Aerosil-R972(g) | - | | - | | - | | 1.50 | | 1.80 | | - | | - | | - | | - | | - | | - | |
| | Aeroxide-P25(g) | - | | - | | - | | - | | - | | 0.60 | | 1.50 | | 1.80 | | - | | - | | - | |
| | Aeroxide-T805(g) | - | | | | | | - | | - | | - | | - | | - | | 1.20 | | 1.50 | | 1.80 | |
| | Glyceryl monoisostearate(g) | 0.06 | | 0.06 | | 0.06 | | 0.06 | | 0.06 | | 0.06 | | 0.06 | | 0.06 | | 0.06 | | 0.06 | | 0.06 | |
| | Pharmaceutical particles(g) | 2.40 | | 2.40 | | 2.40 | | 2.40 | | 2.40 | | 2.40 | | 2.40 | | 2.40 | | 2.40 | | 2.40 | | 2.40 | |
| Solvent | Ethanol(g) | 47.4 | | 46.2 | | 45.9 | | 45.9 | | 45.6 | | 46.8 | | 45.6 | | 45.6 | | 46.2 | | 45.9 | | 45.6 | |
| Inorganic particles wt%(to a water-insoluble polymer) | | 0.0 | | 50.0 | | 62.5 | | 62.5 | | 75.0 | | 25.0 | | 82.5 | | 75.0 | | 50.0 | | 62.5 | | 75.0 | |
| Inorganic particles wt%(to pharmaceutical particles) | | 0.0 | | 50.0 | | 62.5 | | 62.5 | | 75.0 | | 25.0 | | 62.5 | | 75.0 | | 50.0 | | 62.5 | | 75.0 | |
| Pharmaceutical elution rate (%) | | pH1.2 | pH6.8 | pH1.2 | pH6.8 | pH1.2 | pH6.8 | pH1.2 | pH6.8 | pH1.2 | pH6.8 | pH1.2 | pH6.8 | pH1.2 | pH6.8 | pH1.2 | pH6.8 | pH1.2 | pH6.8 | pH1.2 | pH6.8 | pH1.2 | pH6.8 |
| | 10min | 22.2 | 34.6 | 12.1 | 16.2 | 10.4 | 21.1 | 10.0 | 23.6 | 11.3 | 21.6 | 18.3 | 31.5 | 14.4 | 23.3 | 13.2 | 20.6 | 13.3 | 24.9 | 11.7 | 24.7 | 10.0 | 24.6 |
| | 30min | 40.4 | 64.6 | 24.0 | 35.4 | 20.6 | 39.3 | 18.6 | 40.0 | 20.7 | 42.4 | 33.1 | 56.7 | 26.0 | 39.3 | 23.7 | 33.5 | 25.3 | 41.8 | 22.1 | 42.4 | 19.0 | 43.0 |
| | 60min | 61.0 | 93.0 | 36.4 | 56.0 | 32.6 | 56.4 | 28.4 | 60.1 | 31.8 | 61.8 | 48.4 | 80.9 | 40.3 | 54.8 | 37.7 | 46.1 | 35.1 | 61.5 | 32.6 | 59.1 | 30.1 | 56.7 |
| Averaga particle diameter(µm) | | 9.2 | | 8.7 | | 8.8 | | 10.5 | | 12.4 | | 8.8 | | 8.4 | | 8.4 | | 8.6 | | 8.6 | | 8.9 | |

| | | | | | | | | | | | | | | | | | | | | | | | |
|---|---|---|---|---|---|---|---|---|---|---|---|---|---|---|---|---|---|---|---|---|---|---|---|
| Spraying solution amount (5 mL/min), air intake temperature (120°C), and spraying air flow rate (470 L/hr) | | | | | | | | | | | | | | | | | | | | | | | |

As shown in Table 3, it was found that the particulate formulations of Examples 10, 11, and 15 to 17 containing a water-insoluble polymer and inorganic particles subjected to hydrophobization in combination were provided with a strengthened effect particularly on the enteric effect as compared with the particulate formulations of Examples 8, 9, and 12 to 14 containing a water-insoluble polymer and inorganic particles not subjected to hydrophobization in combination.
On the other hand, the particulate formulation of Comparative Example 3 containing no inorganic particles failed to effectively control (suppress) the drug dissolution.
In addition, it was tried to prepare a particulate formulation having a first coating layer and a second coating layer by using the particulate formulation of Example 7 as the drug particles and the second coating solution prepared under the same condition as that in Example 10 except that the solvent was changed to acetone; however, since the HPMCP in the first coating layer was dissolved in acetone and became highly viscous, the second coating solution could not be sprayed.

### (Example 18)

With the composition shown in Table 4, hydroxypropylmethyl cellulose phthalate (HPMCP), hydrophobic lightweight silicic anhydride (Aerosil-R972), glyceryl monoisostearate, stearic acid, and potassium guaiacolsulfonate drug particles were suspended in acetone. The prepared suspension was spray-dried with a spray dryer (Mini Spray Dryer B-290 from BUCHI Labortechnik AG) at a spraying solution amount of 5 mL/min, an air intake temperature of 120°C, and a spraying air flow rate of 470 L/hr to obtain a particulate formulation. The particulate formulation was subjected to the dissolution test in dissolution medium at pH 1.2 and pH 7.2. The results of the test are collectively shown in Table 1.

### (Comparative Example 4)

A particulate formulation was prepared in the same manner as in Example 1, except that no stearic acid was added. The particulate formulation was subjected to the dissolution test in dissolution medium at pH 1.2 and pH 7.2. The composition and results of the test are collectively shown in Table 4.

**[Table 4]**

| Composition | | Comparative Example 4 | | Example 18 | |
|---|---|---|---|---|---|
| Formulation components | HPMCP(g) | 2.40 | | 2.40 | |
| | Aerosil-R972(g) | 1.80 | | 1.80 | |
| | Stearic acid(g) | 0.00 | | 0.30 | |
| | Glyceryl monoisostearate(g) | 0.060 | | 0.060 | |
| Pharmaceutical particles | Potassium guaiacol sulfonate pharmaceutical particles(g) | 2.40 | | 2.40 | |
| Solvent | Acetone(g) | 45.6 | | 45.6 | |
| Pharmaceutical elution rate 60 minutes after starting the test(%) | | pH1.2 | pH7.2 | pH1.2 | pH7.2 |
| | | 47.8 | 79.9 | 48.5 | 100.0 |
| Elution rate ratio between pH7.2 and pH1.2 | | 1.7 | | 2.1 | |
| Average particle diameter of particulate formulations (*µ*m) | | 9.2 | | 9.5 | |

As shown in Table 4, it was found that the particulate formulation of Example 18 containing HPMCP (an enteric polymer) as a water-insoluble polymer and a lipid components in a prescribed weight ratio had an average particle diameter as small as 9.5 µm and could effectively control (suppress) the drug dissolution under a condition of pH 1.2 and could promote drug dissolution under a condition of pH 7.2 and thus, showed a high dissolution rate ratio between pH 7.2 and pH 1.2 and the characteristic as an enteric drug.
On the other hand, the particulate formulation of Comparative Example 4 containing no lipid component failed to effectively promote the drug dissolution under a condition of pH 7.2 as compared with the particulate formulation of Example 18.

### (Example 19)

With the composition shown in Table 5, ethyl cellulose, Eudragit S100, palmitic acid, hydrophobic lightweight silicic anhydride (Aerosil-R972), glyceryl monoisostearate, and potassium guaiacolsulfonate drug particles were suspended in ethanol. The prepared suspension was spray-dried with a spray dryer (Mini Spray Dryer B-290 from BUCHI Labortechnik AG) at a spraying solution amount of 5 mL/min, an air intake temperature of 120°C, and a spraying air flow rate of 470 L/hr to obtain a particulate formulation. The particulate formulation was subjected to the dissolution test in dissolution medium at pH 1.2 and pH 7.2. The results of the test are collectively shown in Table 5.

### (Example 20)

A particulate formulation was prepared in the same manner as in Example 19, except that palmitic acid was changed to stearic acid. The particulate formulation was subjected to the dissolution test in dissolution medium at pH 1.2 and pH 7.2. The composition and results of the test are collectively shown in Table 5.

### (Example 21)

A particulate formulation having a first coating layer and a second coating layer was prepared in the same manner as in Example 20, except that the particulate formulation obtained in Example 18 was used in place of the potassium guaiacolsulfonate drug particles. The particulate formulation was subjected to the dissolution test in dissolution medium at pH 1.2 and pH 7.2. The composition and results of the test are collectively shown in Table 5.

### (Example 22)

A particulate formulation was prepared in the same manner as in Example 20, except that the mixing amount of stearic acid was changed from 0.30 g to 0.60 g. The particulate formulation was subjected to the dissolution test in dissolution medium at pH 1.2 and pH 7.2. The composition and results of the test are collectively shown in Table 5.

### (Example 23)

A particulate formulation was prepared in the same manner as in Example 19, except that palmitic acid was changed to arachidic acid. The particulate formulation was subjected to the dissolution test in dissolution medium at pH 1. 2 and pH 7.2. The composition and results of the test are collectively shown in Table 5.

### (Example 24)

A particulate formulation was prepared in the same manner as in Example 19, except that palmitic acid was changed to behenic acid. The particulate formulation was subjected to the dissolution test in dissolution medium at pH 1.2 and pH 7.2. The composition and results of the test are collectively shown in Table 5.

### (Comparative Example 5)

A particulate formulation was prepared in the same manner as in Example 20, except that the mixing amount of stearic acid was changed from 0.30 g to 0.90 g. The particulate formulation was subjected to the dissolution test in dissolution medium at pH 1.2 and pH 7.2. The composition and results of the test are shown in Table 5.

### (Comparative Example 6)

A particulate formulation was prepared in the same manner as in Example 19, except that palmitic acid was changed to myristic acid. The particulate formulation was subjected to the dissolution test in dissolution medium at pH 1. 2 and pH 7.2. The composition and results of the test are collectively shown in Table 5.

**[Table 5]**

| Composition | | Example 19 | | Example 20 | | Example 21 | | Example 22 | | Example 23 | | Example 24 | | Comparative Example 5 | | Comparative Example 6 | |
|---|---|---|---|---|---|---|---|---|---|---|---|---|---|---|---|---|---|
| Formulation components | Ethyl cellulose(g) | 1.20 | | 1.20 | | 1.20 | | 1.20 | | 1.20 | | 1.20 | | 1.20 | | 1.20 | |
| | Eudragit-S100(g) | 1.20 | | 1.20 | | 1.20 | | 1.20 | | 1.20 | | 1.20 | | 1.20 | | 1.20 | |
| | Aerosil-R972(g) | 1.50 | | 1.50 | | 1.50 | | 1.50 | | 1.50 | | 1.50 | | 1.50 | | 1.50 | |
| | Myristic acid(C14)(g) | - | | - | | - | | - | | - | | - | | - | | 0.30 | |
| | palmitic acid(C16)(g) | 0.30 | | - | | - | | - | | - | | - | | - | | - | |
| | Stearic acid(C18)(g) | - | | 0.30 | | 0.30 | | 0.60 | | - | | - | | 0.90 | | - | |
| | Arachidic acid(C20)(g) | - | | - | | - | | - | | 0.30 | | - | | - | | - | |
| | Behenic acid(C22)(g) | - | | - | | - | | - | | - | | 0.30 | | - | | - | |
| | Glyceryl monoisostearate(g) | 0.060 | | 0.060 | | 0.060 | | 0.060 | | 0.060 | | 0.060 | | 0.060 | | 0.060 | |
| Pharmaceutical particles | Potassium guaiacol sulfonate pharmaceutical particles(g) | 2.40 | | 2.40 | | - | | 2.40 | | 2.40 | | 2.40 | | 2.40 | | 2.40 | |
| | Coated particles(Example 1)(g) | - | | - | | 2.40 | | - | | - | | - | | - | | - | |
| Solvent | Ethanol(g) | 45.6 | | 45.6 | | 45.6 | | 45.3 | | 45.6 | | 45.6 | | 45.0 | | 45.6 | |
| Pharmaceutical elution rate 60 minutes after starting the test(%) | | pH1.2 | pH7.2 | pH1.2 | pH7.2 | pH1.2 | pH7.2 | pH1.2 | pH7.2 | pH1.2 | pH7.2 | pH1.2 | pH7.2 | pH1.2 | pH7.2 | pH 1.2 | pH7.2 |
| | | 40.3 | 99.1 | 29.0 | 100.0 | 14.7 | 80.4 | 43.9 | 94.6 | 43.0 | 88.2 | 22.7 | 46.3 | 58.4 | 80.9 | 64.6 | 65.6 |
| Elution rate ratio between pH7.2 and pH1.2 | | 2.5 | | 3.5 | | 5.5 | | 2.2 | | 2.1 | | 2.0 | | 1.4 | | 1.0 | |
| Average particle diameter of particulate formulations (µm) | | 13.2 | | 9.0 | | 16.6 | | 11.0 | | 11.8 | | 11.0 | | 10.0 | | 11.8 | |

As shown in Table 5, it was found that the particulate formulations of Examples 19 to 24 containing Eudragit S100 (an enteric polymer) and ethyl cellulose (a sustained release polymer) as water-insoluble polymers and a C₁₅ or higher fatty acid as a lipid component in a prescribed weight ratio, had an average particle diameter as small as 9.0 to 16.6 µm and could effectively control (suppress) the drug dissolution under a condition of pH 1.2 and could promote drug dissolution under a condition of pH 7.2 and thus, showed a high dissolution rate ratio between pH 7.2 and pH 1.2 and the characteristic as an enteric drug. Additionally, the particulate formulation of Example 24 using behenic acid of C₂₂ as a lipid component was slightly inferior in the drug dissolution rate under a condition of pH 7.2 as compared with other examples. Based on comparison between Example 20 and Example 21, increase of the average particle diameter and decrease (suppression) of dissolution rate were found by forming a first coating layer and a second coating layer as compared with those in the case of monolayer coating (Example 20) and the particulate formulation with effectively suppressed dissolution was obtained.
On the other hand, the particulate formulation of Comparative Example 5 containing 0. 90 parts by weight of stearic acid as a lipid component and the particulate formulation of Comparative Example 6 containing myristic acid (C₁₄) as a lipid component failed to control (suppress) the drug dissolution under a condition of pH 1.2 and the dissolution rate ratio between pH 7.2 and pH 1.2 was narrowed.

### (Example 25)

With the composition shown in Table 6, ethyl cellulose, Eudragit S100, glyceryl monoisostearate, stearic acid, and Tamsulosin hydrochloride drug particles were suspended in ethanol.
The prepared suspension was spray-dried with a spray dryer (Mini Spray Dryer B-290 from BUCHI Labortechnik AG) at a spraying solution amount of 5 mL/min, an air intake temperature of 120°C, and a spraying air flow rate of 470 L/hr to obtain a particulate formulation. The particulate formulation was subjected to the dissolution test in dissolution medium at pH 1.2 and pH 6.8. The results of the test are collectively shown in Table 6.

### (Example 26)

A particulate formulation was prepared in the same manner as in Example 25, except that hydrophobic lightweight silicic anhydride (Aerosil-R972) was further added. The particulate formulation was subjected to the dissolution test in dissolution medium at pH 1.2 and pH 6.8. The composition and results of the test are collectively shown in Table 6.

### (Comparative Example 7)

A particulate formulation was prepared in the same manner as in Example 25, except that no stearic acid was added. The particulate formulation was subjected to the dissolution test in dissolution medium at pH 1.2 and pH 6.8. The composition and results of the test are collectively shown in Table 6.

### (Comparative Example 8)

A particulate formulation was prepared in the same manner as in Example 26, except that no stearic acid was added. The particulate formulation was subjected to the dissolution test in dissolution medium at pH 1.2 and pH 6.8. The composition and results of the test are collectively shown in Table 6.

### (Comparative Example 9)

Tamsulosin hydrochloride drug particles physically milled by a jet mill were subjected to the dissolution test with dissolution medium at pH 1.2 and pH 6.8. The results of the test are collectively shown in Table 6.

**[Table 6]**

| Composition | | Example 25 | | Example 26 | | Comparative Example 7 | | Comparative Example 8 | | Comparative Example 9 | |
|---|---|---|---|---|---|---|---|---|---|---|---|
| Formulation components | Ethyl cellulose(g) | 1.20 | | 1.20 | | 1.20 | | 1.20 | | Tamusulosin hydrochloride pharmaceutical particles (without coating) | |
| | Eudragit-S 100(g) | 1.20 | | 1.20 | | 1.20 | | 1.20 | | | |
| | Aerosil-R972(g) | - | | 0.60 | | - | | 0.60 | | | |
| | Stearic acid(C18)(g) | 0.30 | | 0.30 | | - | | - | | | |
| | Glyceryl monoisostearate(g) | 0.060 | | 0.060 | | 0.060 | | 0.060 | | | |
| Pharmaceutical particles | Tamusulosin hydrochloride pharmaceutical particles(g) | 2.40 | | 2.40 | | 2.40 | | 2.40 | | | |
| Solvent | Ethanol(g) | 45.6 | | 45.6 | | 45.6 | | 45.6 | | | |
| Pharmaceutical elution rate 60 minutes after starting the test(%) | | pH 1.2 | pH6.8 | pH1.2 | pH6.8 | pH 1.2 | pH6.8 | pH1.2 | pH6.8 | pH1.2 | pH6.8 |
| | | 2.6 | 83.6 | 1.5 | 65.8 | 3.7 | 68.2 | 3.8 | 20.6 | 36.7 | 88.4 |
| Elution rate ratio between pH6.8 and pH1.2 | | 32.2 | | 43.9 | | 18.4 | | 5.4 | | 2.4 | |
| Average particle diameter of particulate formulations(µm) | | 10.6 | | 10.9 | | 11.1 | | 10.6 | | 2.7 | |

As shown in Table 6, the drug particles of Comparative Example 9 physically milled by a jet mill showed high drug dissolution rate under a condition of pH 1.2 and pH 6.8.
It was found that the particulate formulation of Example 25 containing Eudragit S100 (an enteric polymer) and ethyl cellulose (a sustained release polymer) as water-insoluble polymers and C₁₈ stearic acid as a lipid component in a prescribed weight ratio had an average particle diameter as small as 10.6 µm and could effectively control (suppress) the drug dissolution under a condition of pH 1.2 and could promote drug dissolution under a condition of pH 6.8 and thus, showed a high dissolution rate ratio between pH 6.8 and pH 1.2 and the characteristic as an enteric drug. On the other hand, the particulate formulation of Comparative Example 7 containing no lipid component failed to effectively promote the drug dissolution under a condition of pH 6.8 as compared with the particulate formulation of Example 25 and the dissolution rate ratio between pH 6.8 and pH 1.2 became low.

In the case of Tamsulosin hydrochloride, being different from that in the case of potassium guaiacolsulfonate, the dissolution of the drug could be effectively controlled (suppressed) under a condition of pH 1.2 while dissolution of the drug was promoted under a condition of pH 6.8 without using hydrophobic lightweight silicic anhydride. Further, the drug of the drug could be effectively controlled (suppressed) under a condition of pH 1.2 with a monolayer coating without the need of formation of bilayer coating as in the case of using potassium guaiacolsulfonate.

It was found that the particulate formulation of Example 26 containing Eudragit S100 (an enteric polymer) and ethyl cellulose (a sustained release polymer) as water-insoluble polymers, C₁₈ stearic acid as a lipid component, and hydrophobic lightweight silicic anhydride in a prescribed weight ratio had an average particle diameter as small as 10.9 µm and could effectively control (suppress) the drug dissolution under a condition of pH 1.2 and could promote drug dissolution under a condition of pH 6.8 and thus, showed a high dissolution rate ratio between pH 6.8 and pH 1.2 and the characteristic as an enteric drug. On the other hand, the particulate formulation of Comparative Example 8 containing no lipid component failed to effectively promote the drug dissolution under a condition of pH 6.8 as compared with the particulate formulation of Example 26 and the dissolution rate ratio between pH 6.8 and pH 1.2 became low.

It was found that, as compared with the particulate formulation of Example 26 containing 0.60 parts by weight of hydrophobic lightweight silicic anhydride, the particulate formulation of Example 25 containing no hydrophobic lightweight silicic anhydride could control (suppress) the drug dissolution under a condition of pH 6.8 while effectively controlling (suppressing) the drug dissolution under a condition of pH 1.2. As described above, addition of hydrophobic lightweight silicic anhydride enabled dissolution control of the drug under a condition of pH 6.8 while effectively controlling (suppressing) the drug dissolution under a condition of pH 1.2.

The dissolution of the drug could be freely controlled in an acidic condition and a neutral condition by changing the number of times of coating, the type and addition amount of the water-insoluble polymer, the addition amount of hydrophobic lightweight silicic anhydride, and the addition amount of the lipid component in accordance with the physical properties of the drug such as solubility.

### INDUSTRIAL APPLICABILITY

In the present invention, the drug particles can be effectively coated and dissolution of the drug can be effectively suppressed even in the case the diameter of the drug particles is small by coating the drug particles with a first coating layer containing a water-insoluble polymer, inorganic particles, and/or a lipid component.
Accordingly, the particulate formulation of the present invention can be administered as it is in form of a fine granule to a living body and also can be administered after being formed into various kinds of formulations.

## Claims

1. A particulate formulation containing drug particles and a first coating layer coating said drug particles, **characterized in that** said first coating layer contains a water-insoluble polymer, inorganic particles, and/or a lipid component and said lipid component contains a C₁₅ or higher fatty acid.

2. The particulate formulation according to claim 1, wherein the water-insoluble polymer contains an enteric polymer.

3. The particulate formulation according to claim 1, wherein the average particle diameter of the inorganic particles is 1 to 1000 nm.

4. The particulate formulation according to claim 1, wherein the inorganic particles are of silica and/or titanium dioxide.

5. The particulate formulation according to claim 1, wherein the inorganic particles are subjected to hydrophobization.

6. The particulate formulation according to claim 1, wherein the inorganic particles are contained in an amount of 0.5 to 2.4 parts by weight to 2.4 parts by weight of the water-insoluble polymer.

7. The particulate formulation according to claim 1, wherein the inorganic particles are contained in an amount of 0.5 to 2.4 parts by weight to 2.4 parts by weight of the drug particles.

8. The particulate formulation according to claim 1, wherein the lipid component contains a C₁₅ to C₂₁ fatty acid.

9. The particulate formulation according to claim 8, wherein the C₁₅ to C₂₁ fatty acid is stearic acid.

10. The particulate formulation according to claim 1, wherein the lipid component is contained in an amount of 0.1 to 0.8 parts by weight to 2.4 parts by weight of the water-insoluble polymer.

11. The particulate formulation according to claim 1, further containing a second coating layer coating the first coating layer.

12. The particulate formulation according to claim 11, wherein the first coating layer contains a cellulose enteric polymer and the second coating layer contains a water-insoluble polymer different from said cellulose enteric polymer.

13. The particulate formulation according to claim 12, wherein the cellulose enteric polymer contains hydroxypropylmethyl cellulose phthalate (HPMCP) and/or hydroxypropylmethyl cellulose acetate succinate (HPMCAS).

14. The particulate formulation according to claim 12, wherein the water-insoluble polymer different from the cellulose enteric polymer contains at least one kind selected from the group consisting of ethyl cellulose, a methacrylic acid-methyl acrylate copolymer, a methyl methacrylate-butyl methacrylate-dimethylaminoethyl methacrylate copolymer, polyvinyl acetal diethylaminoacetate, an ethyl acrylate-methyl methacrylate-chlorotrimethylammonium ethyl methacrylate copolymer, and an ethyl acrylate-methyl methacrylate copolymer.

15. The particulate formulation according to claim 1, having an average particle diameter of 0.1 to 200 µm.

16. A method for producing a particulate formulation, comprising the steps of:
forming a first coating layer on drug particles by dissolving and/or suspending said drug particles in a first coating solution containing a cellulose enteric polymer and carrying out a spray drying method; and
forming a second coating layer by suspending said drug particles coated with said first coating layer in a second coating solution containing a water-insoluble polymer different from said cellulose enteric polymer and carrying out a spray drying method.
Claim 17. The method for producing a particulate formulation according to claim 16, wherein the first coasting solution contains a first polar solvent.
Claim 18. The method for producing a particulate formulation according to claim 17, wherein the first polar solvent is acetone.
Claim 19. The method for producing a particulate formulation according to claim 16, wherein the second coating solution contains a second polar solvent different from the first polar solvent.
Claim 20. The method for producing a particulate formulation according to claim 19, wherein the second polar solvent is ethanol.
